# EUROPÄISCHE PATENTSCHRIFT

(11) **EP 0 716 079 B1**
(45) Veröffentlichungstag und Bekanntmachung des Hinweises auf die Patenterteilung: **10.02.1999**
(21) Anmeldenummer: 95118607.1
(22) Anmeldetag: 27.11.1995
(51) Int. Cl.: C07C 263/10

(54) **Kontinuierliches Verfahren zur Herstellung von organischen Isocyanaten**
Continuous process for the manufacture of organic isocyanates
Procédé en continu de préparation d'isocyanates organiques

(30) Priorität: 08.12.1994 DE 4443642
(43) Veröffentlichungstag der Anmeldung: 12.06.1996
(73) Patentinhaber: BAYER AG, 51368 Leverkusen (DE)
(72) Erfinder: Bankwitz, Robert, Dr., D-47906 Kempen (DE); Gebauer, Herbert, Dr., D-47829 Krefeld (DE); König, Christian, Dr., D-41564 Kaarst (DE); Waldau, Eckart, Dr., D-40597 Düsseldorf (DE)

(56) Entgegenhaltungen:
- EP-A- 0 314 985
- EP-A- 0 322 647
- DE-A- 2 058 032
- DE-A- 2 112 181
- US-A- 2 875 225

## Beschreibung

Die vorliegende Erfindung betrifft ein kontinuierliches Verfahren zur Herstellung von organischen Isocyanaten durch Umsetzung von primären organischen Aminen mit Phosgen in einem organischen Lösungsmittel bei Temperaturen von 60 bis 100°C, wobei das Reaktionsgemisch mittels einer Blasensäule umgewälzt wird.

Die kontinuierliche Herstellung von organischen Isocyanaten durch Reaktion von primären organischen Aminen mit Phosgen in einem organischen Lösungsmittel ist vielfach beschrieben und wird im großtechnischen Maßstab durchgeführt (s. z.B. Ullmanns Enzyklopädie der Technischen Chemie, 4. Auflage, Band 13; Kunststoffhandbuch, Band 7 (Polyurethane) 3., neubearbeitete Auflage, Carl Hanser Verlag, München-Wien, S. 76 (1993)). Der Reaktionsablauf wird meist in zwei Temperaturstufen getrennt, die Kalt- und Heißphosgenierung. In der ersten Stufe wird das Amin mit Phosgen in schneller exothermer Reaktion bei tiefen Temperaturen zu einem Gemisch von Carbamidsäurechlorid und Aminhydrochlorid umgesetzt. Dieses Reaktionsgemisch wird anschließend bei hohen Temperaturen "durchphosgeniert", bis die Gasentwicklung beendet ist. Eine befriedigende Ausbeute wird bei diesem Verfahren erreicht durch hohen Phosgenüberschuß, durch hohe Verdünnung der Reaktionspartner mit einem organischen Lösungsmittel sowie durch die zweistufige Reaktionsführung (Kalt- und Heißphosgenierung).

Nachteilig bei diesem Verfahren ist der hohe Energieverbrauch, der durch die Wiedergewinnung und Reinigung des Lösungsmittels und des Phosgenüberschusses erforderlich wird. Es hat daher nicht an Versuchen gefehlt, das Phosgenierungsverfahren zu verbessern.

So sind in der Patentliteratur eine Reihe von Verfahren beschrieben, die zum Ziel haben, eine sehr wirksame Durchmischung der zu Reaktion gebrachten Ausgangskomponenten - primäres Amin und Phosgen - zu gewährleisten, wobei Kreislaufapparate und/oder dynamische oder statische Mischer zur Anwendung kommen. Dadurch kann bei gleichbleibender Ausbeute in höherer Konzentration und mit geringerem Phosgenüberschuß gearbeitet werden. So beschreibt die Deutsche Offenlegungsschrift 2 212 181 ein Verfahren, bei dem eine intensive Mischung dadurch erreicht wird, daß das flüssige Reaktionsgemisch zusammen mit den gasförmigen Reaktionsprodukten im Gleichstrom über eine Füllkörpersäule mit einer solchen Geschwindigkeit im Kreis gepumpt wird, daß in der Füllkörpersäule eine sogenannte Übergangsstörmung (Transition Flow) mit turbulenten Eigenschaften entsteht. In der Deutschen Offenlegungsschrift 2 624 285 wird ein Verfahren beschrieben, bei dem das Reaktionsgemisch zusammen mit flüssigem Phosgen über eine Treibstrahldüse im Kreis gepumpt wird, wobei eine Lösung des Amins dieser Düse so zugeführt wird, daß eine Reaktions- und Mischzone hoher Energiedissipationsdichte entsteht. Ein weiteres Kreislaufverfahren ist in der Deutschen Offenlegungsschrift 3 212 510 beschrieben. Hierbei wird unter Druck bei einer Temperatur gearbeitet, bei der 30 bis 70 % des im ersten Reaktionsschritt gebildeten Carbamidsäurechlorids in Isocyanat gespalten ist, so daß ein Gemisch mit geringer Viskosität ensteht, das mit hoher Geschwindigkeit umgepumpt werden kann. Im US Patent 3 781 320 wird eine Kreislaufapparatur zur Herstellung von organischen Isocyanaten aus primären Aminen und Phosgen beschrieben, bei der ein Mischaggregat mit hoherer Scherwirkung Anwendung findet.

Die Durchführung der Phosgenierung von Aminen in einer Kreislaufapparatur wird in der Deutschen Patentschrift 1 037 444 beschrieben. Dabei findet die Vermischung der Reaktionspartner oberhalb der Zersetzungstemperatur des Carbaminsäurechlorids unter Druck statt. Da die Abtrennung von Chlorwasserstoff und überschüssigem Phosgen bei niedrigerem Druck erfolgt, wird eine Pumpe mit entsprechender Förderhöhe eingesetzt. Einige der bisher bekannten Verfahren mit Kreislaufapparaturen haben aber den Nachteil, daß bei Temperaturen gearbeitet wird, bei denen das im ersten Reaktionsschritt gebildetete Carbamidsäurechlorid zum überwiegenden Teil oder vollständig in Isocyanat und Chlorwasserstoff gespalten ist und bei denen bei der Reaktion des Amins das freie Isocynat so stark in Konkurrenz zum Phosgen tritt, daß Harnstoffe enstehen, die nicht mehr vollständig zu Isocyanat umgesetzt werden, sondern zu Nebenprodukten führen, die die Ausbeute schmälern bzw. die Produktqualität beeinträchtigen.

Bei anderen Verfahren wird versucht, die erwünschte intensive Durchmischung durch dynamische oder statistische Mischer in einem Durchgang, d.h. ohne Kreislauf zu erreichen. So beschreibt die Deutsche Offenlegungsschrift 2 153 268 die Reaktion von primären Aminen mit Phosgen in organischen Lösungsmitteln in mehrstufen Kreiselpumpen und die Deutsche Offenlegungsschrift 3 121 036 dieselbe Reaktion in einer Glattstrahldüse.

Ein anderer Weg, wenigstens den eingesetzten Phosgenüberschuß zu verringern, ist die Phosgenierung unter Druck. Um eine wirtschaftliche Raum-Zeit-Ausbeute zu gewährleisten, arbeiten alle großtechnischen Verfahren zur Herstellung von organischen Isocyanaten durch Phosgenierung von primären Aminen bei erhöhten Temperaturen. Dabei verringerte sich die Löslichkeit des Phosgens im Reaktionsgemisch und damit der wirksame Phosgenüberschuß. Dem wirkt ein erhöhter Reaktordruck entgegen. Abgesehen davon, daß die für eine gute Ausbeute notwendige hohe Verdünnung bei diesen Verfahren nicht verringert wird, sind großtechnische Druckapparaturen sehr teuer und wegen der hohen Giftigkeit des Phosgens problematisch.

Die Durchführung der Phosgenierreaktion unter Druck hat den Nachteil, daß neben der Phosgenkonzentration auch die HCl-Konzentration entsprechend dem Henry'-schen Gesetzt erhöht wird und dadurch die Bildung von Aminhydrochlorid begünstigt wird. Dadurch wird der Vorteil der höhereren Phosgenkonzentration bezüglich der Reaktionsgeschwindigkeit ganz oder teilweise aufgehoben, da die Reaktion von Aminhydrochlorid mit Phosgen der geschwindigkeitsbestimmende Schritt jedes Phosgenierungsverfahrens zur Herstellung von Isocyanaten ist.

Alle bisher bekannten Verfahren zur Umsetzung von primären Aminen mit Phosgen in Kreislaufapparaturen haben zudem den Nachteil, daß für den Kreislauf Umwälzpumpen erforderlich sind.

Aufgabe der vorliegenden Erfindung ist es daher, die genannten Nachteile der bislang bekannten Verfahren zu vermeiden. Das erfindungsgemäße Verfahren erlaubt es, aus primären Aminen durch Umsetzung mit Phosgen in hoher Ausbeute organische Isocyanate herzustellen, wobei mit einer konzentrierten Amin-Lösung gearbeitet werden kann, der Phosgenüberschuß gering gehalten, der Energieverbrauch drastisch reduziert und keine Umwälzpumpe zum Umpumpen des Reaktionsgemisches benötigt wird.

Gegenstand der vorliegenden Erfindung ist daher ein Verfahren zur kontinuierlichen Herstellung von organischen Isocyanaten durch Umsetzung von primären organischen Aminen mit Phosgen in einem organischen Lösungsmittel, das dadurch gekennzeichnet ist, daß man die Reaktionsmischung bei einer Temperatur von 60 bis 100°C mittels einer Blasensäule umwälzt, wobei das Verhältnis der Menge an umgewälzter Reaktionsmischung, bezogen auf die Gesamtmenge der Reaktionsprodukte des eingesetzten Amins, zu der Menge des zugeführten Amins 100:1 bis 5:1, bevorzugt 80:1 bis 10:1, beträgt.

Als primäre organische Amine können in das erfindungsgemäße Verfahren beliebige mono- oder polyfunktionelle, aliphatische, cycloaliphatische oder aromatische Amine, wie Toluylendiamin, Diaminodiphenylmethan, 1,5 Diaminonaphtalin, chlorierte Aniline, wie 3,4 Dichloranilin, Triphenylmethantriamin, Anilin und Derivate, insbesondere Toluylendiamin oder Diaminodiphenylmethan eingesetzt werden. Die Amine können sowohl als reine Isomere oder als Isomerengemisch eingesetzt werden.

Bei dem erfindungsgemäßen Verfahren werden die einzusetzenden Amine in Lösung dem Reaktionsgemisch zudosiert. Üblicherweise werden die primären organischen Amine in einer 10 bis 50 gew.-%igen Lösung, bevorzugt 20 bis 40 gew.-%igen Lösung, in einem organischen Lösungsmittel eingesetzt.

Als organische, inerte Lösungsmittel können die für das Phosgenierungsverfahren bekannten organischen Lösungsmittel eingesetzt werden, insbesondere eignen sich Chlorbenzol und/oder o-Dichlorbenzol.

Bei der Zugabe der aminischen Lösung zu dem Reaktionsgemisch, das im wesentlichen besteht aus organischem Lösungsmittel, Aminhydrochlorid, Carbamidsäurechlorid, Isocyanat und Phosgen, hat es sich als vorteilhaft erwiesen, daß die Amin-lösung mittels einer Düse in der Strömungsrichtung der umgewälzten Reaktionsmischung mit einer Düsenaustrittsgeschwindigkeit von 5 bis 50 m/s., bevorzugt 20 bis 40 m/s, in die Reaktionsmischung eingespritzt wird.

Nach dem erfindungsgemäßen Verfahren ist es möglich, daß Phosgen in gasförmiger Form oder gelöst in den zuvor genannten inerten organischen Lösungsmittel dem Reaktionsgemisch zu zudosieren. Die Menge an Phosgen beträgt bei dem erfindungsgemäßen Verfahren 110 bis 300 %, bevorzugt 150 bis 250 %, bezogen auf die zur Umsetzung des Amins stöchiometrisch erforderliche Menge.

Das erfindungsgemäße Verfahren wird so gestaltet, daß in einem Behältnis das Reaktionsgemisch mittels einer Blasensäule, die in Folge der Reaktion von Amin mit in der Reaktionsmischung gelösten Phosgen durch gebildeten Chlorwasserstoff und überschüssiges Phosgen entsteht, umgewälzt wird. Dabei ist es wichtig, daß das Verhältnis der Menge an umgewälzter Reaktionsmischung, bezogen auf die Gesamtmenge der Reaktionsprodukte des eingesetzten Amins (Aminhydrochlorid, Carbamidsäurechlorid, Isocyanat), zu der Menge des zugeführten Amins 100:1 bis 5:1, bevorzugt 80:1 bis 10:1, beträgt.

Aus verfahrensökönomischen Gründen ist es von Vorteil, wenn man die Phosgenierung der Amine zu den entsprechenden Isocyanaten nicht in einer Stufe durchführt, sondern in einem zusätzlichen Verfahrensabschnitt, das in der Reaktionsmischung vorhandene Aminhydrochlorid mit Phosgen umsetzt, wobei diese Umsetzung unterhalb der für die vollständige Spaltung von Carbamidsäurechlorid erforderlichen Temperatur durchgeführt wird, und anschließend das entstehende Carbamidsäurechlorid in Gegenwart von Phosgen zu entsprechenden Isocyanaten spaltet.

Die Verweilzeiten des Reaktionsgemisches in dem Reaktionsbehälter betragen üblicherweise etwa 15 min bis 4 Stunden, wobei bei der zuletzt geschilderten Verfahrensvariante, die Verweilzeit in dem Reaktionsbehälter beträchtlich verküzt werden kann, wenn man, wie erwähnt, in einem zusätzlichen Verfahrensabschnitt im Reaktionsgemisch vorhandenes Aminhydrochlorid mit Phosgen weiter umsetzt.

Das erfindungsgemäße Verfahren wird bevorzugt in einem Schleifenreaktor durchgeführt, wobei der Gaslift mit der Blasensäule innerhalb oder außerhalb des Reaktionsbehälters (Reaktorturm) angeordnet ist. Selbstverständlich kann das erfindungsgemäße Verfahren auch in anders konstruierten Reaktoren durchgeführt werden, wobei bei deren Konstruktion darauf zu achten ist, daß die Reaktionsmischung mittels einer Blasensäule umgewälzt wird und ein bestimmtes Verhältnis von umgewälzter Reaktionsmischung zu zugeführter Menge an organischen Aminen eingehalten wird.

Das erfindungsgemäße Verfahren wird in vorteilhafter Weise bei leicht erniedrigten Druck bis leicht erhöhten Druck durchgeführt. Im allgemeinen arbeitet man im Druckbereich von 0,5 bar bis 5 bar, bevorzugt 1 bar bis 3 bar.

Die Aufarbeitung des erhaltenen Reaktionsgemisches geschieht auf übliche Art und Weise, in dem man beispielsweise das Reaktionsgemisch von überschüssigem Phosgen befreit, das organische Lösungsmittel durch Destillation wiedergewinnt und zur Reindarstellung der Isocyanate das Reaktionsgemisch gegebenenfalls mehrfach destilliert. Das konzentierte, lösungsmittelfreie Rohprodukt kann dann direkt zu Polyurethanen weiterverarbeitet werden.

Die Ausbeuten an organischen Isocyanaten nach dem erfindungsgemäßen Verfahren liegen im allgemeinen bei 90 bis 99 % der Theorie.

### Beispiel

Verwendet wurde ein Schleifenreaktor, bestehend aus einem 2 m hohen Turm von 50 mm Durchmesser mit einem Kopf von 80 mm Durchmesser zur Gasabscheidung, einem äußeren Umwälzrohr von 15 mm Durchmesser und einem Nachreaktionsrohr von 15 mm Durchmesser, welches über die volle Höhe zentrisch im Turm angeordnet war und im Reaktorkopf herausgeführt war und in einem Dünnschichtverdampfer von 40 mm Durchmesser und 0,5 m Höhe führte. Weiterhin war auf dem Reaktorkopf ein Brüdenabgasrohr angebracht, welches ebenfalls oben auf den Dünnschichtverdampfer führte. Das Umwälzrohr hatte unten in Höhe des Turmbodens über eine Länge von 50 mm eine Verjüngung von 6 mm, in die von unten, mit Öffnung nach oben, ein Kapillarrohr von 0,17 mm hineinragte. Am oberen Ende war um die Verjüngung des Umwälzrohres ein Ringspalt zur Gaseinspeisung angeordnet.

Das Umwälzrohr war oben tangential in den Reaktorkopf eingeführt. der Schleifenreaktor hatte incl. Nachreaktionsteil ein nutzbares Volumen von 5,4 l. Der Turm war mit einem Dämmantel und das Umwälzrohr mit einem Heizmantel umgeben.

In den Schleifenreaktor wurde zum Anfahren 3 %ige Phosgenlösung in Monochlorbenzol, welche bereits Reaktionstemperatur von 80°C hatte, vorgelegt. Die Anfahrzeit betrug mindestens die vierfache Verweilzeit. Der Dünnschichtverdampfer wurde mit einer Manteltemperatur von 150°C betrieben. In die Gaseinspeisung wurde ein Phosgenstrom von 1 800 g/h eingeleitet. Diaminodiphenylmethan mit einer Viskosität von 88 mPa.s (in einem Gemisch, wie es durch Kondensation von Anilin und Formaldehyd entstand) wurde in Mischung mit drei Massenteilen Monochlorbenzol mit einer Düsenaustrittsgeschwindigkeit von etwa 40 m/s durch das Kapillarrohr mittels einer Kolbendosierpumpe in den Schleifenraktor eingespritzt. Der durch die Blasensäule, welche durch freigesetztes Chlorwasserstoffgas und überschüssiges Phosgen in dem Umwälzrohr entstand, umgewälzte Massenstrom der Reaktionsmischung betrug 180 kg/h. Bei diesem Verfahren wurden pro Stunde je Liter nutzbares Reaktorvolumen nach der Lösungsmittelabtrennung 208 g Gemisch des 4,4'-Diisocyanatodiphenylmehans mit seinen Isomeren und Homologen mit einer Viskosität von 71 mPa.s und einem NCO-Wert von 32,1 Gew.-% erhalten.

## Patentansprüche

1. Verfahren zur kontinuierlichen Herstellung von organischen Isocyanaten durch Umsetzung von primären organischen Aminen mit Phosgen in Gegenwart eines inerten, organischen Lösungsmittels, dadurch gekennzeichnet, daß man das Reaktionsgemisch bei einer Temperatur von 60 bis 100°C mittels einer Blasensäule umwälzt, wobei das Verhältnis der Menge an umgewälzter Reaktionsmischung, bezogen auf die Gesamtmenge der Reaktionsprodukte des eingesetzten Amins, zur Menge des zugeführten Amins 100:1 bis 5:1 beträgt.

2. Verfahren nach Anspruch 1, dadurch gekennzeichnet, daß man die Umsetzung in einem Schleifenreaktor durchführt, wobei die Umwälzung des Reaktionsgemisches mittels Blasensäule innerhalb oder außerhalb des Reaktors erfolgt.

3. Verfahren nach Anspruch 1, dadurch gekennzeichnet, daß man in einem zusätzlichen Verfahrensschritt das in der Reaktionsmischung vorhandene Aminhydrochlorid mit Phosgen umsetzt, wobei die Umsetzung bei einer Temperatur durchgeführt wird, die unterhalb der für die vollständige Spaltung von Carbamidsäurechlorid erforderlichen Temperatur liegt.

4. Verfahren nach Anspruch 1, dadurch gekennzeichnet, daß man das primäre organische Amin als 10 bis 50 gew.-%ige organische Lösung einsetzt.

5. Verfahren nach Anspruch 1, dadurch gekennzeichnet, daß man die organische Aminlösung mittels einer Düse in der Strömungsrichtung der umgepumpten Reaktionsmischung mit einer Düsenaustrittsgeschwindigkeit von 5 m/s bis 50 m/s in die Reaktionsmischung einspritzt.

## Claims

1. Process for the continuous preparation of organic isocyanates by reacting primary organic amines with phosgene in the presence of an inert, organic solvent, characterised in that the reaction mixture is circulated by means of a bubble column at a temperature of from 60 to 100°C, with the ratio of the quantity of circulated reaction mixture, referred to the total quantity of the reaction products of the used amine, to the quantity of the amine supplied, being from 100:1 to 5:1.

2. Process according to claim 1, characterised in that the reaction is carried out in a loop reactor, with the circulation of the reaction mixture being carried out by means of a bubble column inside or outside the reactor.

3. Process according to claim 1, characterised in that the amine hydrochloride present in the reaction mixture is reacted with phosgene in a further processing step, with the reaction being carried out at a temperature below the temperature required for the complete decomposition of carbamyl chloride.

4. Process according to claim 1, characterised in that the primary organic amine is used as a 10 to 50 wt.% organic solution.

5. Process according to claim 1, characterised in that the amine solution is injected into the reaction mixture by means of a nozzle in the direction of flow of the circulated reaction mixture at a nozzle discharge velocity of from 5 m/s to 50 m/s.

## Revendications

1. Procédé pour la préparation en continu d'isocyanates organiques par mise en réaction d'amines organiques primaires avec du phosgène en présence d'un solvant organique inerte, caractérisé en ce qu'on fait circuler le mélange réactionnel à une température de 60 à 100°C au moyen d'une colonne à bulles, le rapport de la quantité du mélange réactionnel mis en circulation, rapportée à la quantité totale des produits réactionnels de l'amine mise en oeuvre, à la quantité de l'amine ajoutée s'élève de 100:1 à 5:1.

2. Procédé selon la revendication 1, caractérisé en ce qu'on effectue la mise en réaction dans un réacteur en boucle, la mise en circulation du mélange réactionnel étant réalisée à l'aide d'une colonne à bulles à l'intérieur ou à l'extérieur du réacteur.

3. Procédé selon la revendication 1, caractérisé en ce que, dans une étape opératoire supplémentaire, on fait réagir avec du phosgène le chlorhydrate d'amine présent dans le mélange réactionnel, la mise en réaction étant réalisée à une température qui est inférieure à la température requise pour la décomposition complète du chlorure d'acide carbamique.

4. Procédé selon la revendication 1, caractérisé en ce qu'on met en oeuvre l'amine organique primaire sous forme d'une solution organique à raison de 10 à 50% en poids.

5. Procédé selon la revendication 1, caractérisé en ce qu'on introduit par injection dans le mélange réactionnel la solution d'amine organique au moyen d'une buse dans la direction d'écoulement du mélange réactionnel transvasé par pompage, avec une vitesse de sortie de la buse de 5 m/s à 50 m/s.
